# EUROPEAN PATENT SPECIFICATION

(11) **EP 0 883 408 B1**
(45) Date of publication and mention of the grant of the patent: **04.12.2002**
(21) Application number: 97905494.7
(22) Date of filing: 27.02.1997
(51) Int. Cl.: A61L 2/18

(54) **OPHTHALMOLOGICALLY USEFUL COMPOSITION, PRODUCTS CONTAINING THE COMPOSITION AND PROCESS FOR DISINFECTING AND/OR CLEANING CONTACT LENSES**
OPTHALMOLOGISCHE ZUSAMMENSETZUNG, PRODUKTEN DIE SIE ENTHALTEN, UND VERFAHREN ZUR DESINFEKTION UND/ODER REINIGUNG VON KONTAKTLINSEN
COMPOSITIONS A USAGE OPHTALMOLOGIQUE, PRODUITS CONTENANT CETTE COMPOSITION ET PROCESSUS DE DESINFECTION ET/OU DE NETTOYAGE DE LENTILLES DE CONTACT

(30) Priority: 29.02.1996 NL 1002484
(43) Date of publication of application: 16.12.1998
(73) Proprietor: de Bruijn, Christianus Hendrikus Mattias Marie, 48683 Ahaus (DE)
(72) Inventor: de Bruijn, Christianus Hendrikus Mattias Marie, 48683 Ahaus (DE)
(74) Representative: Van kan, Johan Joseph Hubert, Ir.
(86) International application number: NL9700092
(87) International publication number: WO97031658

(56) References cited:
- WO-A-94/00160
- US-A- 4 367 157
- US-A- 5 425 944

## Description

### Background of the invention.

This invention relates to ophthalmologically useful compositions, eye drops, artificial tears, eye care products containing the compositions, and a process for desinfecting and/or cleaning contact lenses. The compositions contain natural substances with remarkable antimicrobial activity, these substances being based on bioflavonoid compounds, either in combination with ascorbic acid or not.

An object in this invention is to provide compositions and methods for the disinfecting/cleaning of contact lenses and the preservation of eye care products. Another object of the invention is to provide compositions and methods for the preservation of eye lubricants and solutions containing pharmacologically relevant compounds.

Contact lens care solutions include products for disinfection, cleaning, wetting and conditioning of the contact lens material. Disinfection solutions contain an amount of antimicrobial activity, which allows the killing of microorganisms occurring in association with contact lenses, within a convenient period of time (varying from 10 minutes to an overnight period). Preserved contact lens care solutions for storing, wetting or conditioning usually contain a lower amount of antimicrobial substance(s) which prevent microbial growth during a certain period of time after the container with the contact lens care solution has been opened.

Several chemical compounds are presently used for disinfection and/or preservation of contact lens care products, including benzalkoniumchloride, thiomersal, sorbic acid and quaternary ammonium compounds, such as polyhexamethylenebiguanides (PHMB); depending on the individual lens hygiene and individual reactivity, the lens material sometimes retains disinfection compounds, which might lead to discomfort and eye irritations.

The so-called All-in-One disinfection/cleaning solutions contain both disinfection and cleaning compounds (e.g. non-ionic detergents). After the disinfection/cleaning step, the contact lenses can be directly applied to the eye.

Contact lenses can also be cleaned by means of a two-step system, such as described in the European Patent Application 0 082 798, the lenses being cleaned with a substance having antimicrobial activity, such as hydrogen peroxide and subsequently in a second step the residual hydrogen peroxide on the lenses is removed. In EP-A-0 456 467 a composition is described for cleaning of contact lenses, in which the cleaning is carried out in one step by immersing the contact lenses in a composition containing a substance with antimicrobical activity which does not need to be removed from the contact lenses. After immersing the contaminated contact lense into such a composition, there is no need to remove the residual substance, because it has no harmful or irritating effects on the eye. The composition described in EP-A 0 456 467 contains an opthalmologically acceptable enzyme, a nitrogen containing antimicrobial polymer and a complex forming agent. As a complexing agent, citric acid, EDTA, acetic acid or a pharmaceutically acceptable salt thereof is used. The enzyme is stored separately from the other ingredients and the two components have to be mixed prior to use, which is inconvenient and, therefore, a disadvantage.

In International Patent Application WO 94/00160, ophthalmic compositions containing grapefruit seed extract as a preservative or disinfectant, and methods for preserving and using such compositions in caring for contact lenses are described. However, bioflavonoids are not mentioned.

The composition according to the present invention is an all-in-one contact lens care composition which does not need any pretreatment or mixing step before use, and which provides a long-lasting antimicrobial activity, while being more compatible with the eye and having less side effects than the prior art compositions.

### Summary of the invention.

According to the present invention, ophthalmologically useful compositions, which are suitable for the disinfecting and/or cleaning of contact lenses, contain substances with an antimicrobial activity, characterised in that the substances with antimicrobial activity belong to the group of bioflavonoids. The effects of antimicrobial compositions containing bioflavonoids can be enhanced with ascorbic acid.

New compositions for the disinfection of contact lenses, for the preservation of liquid contact lens care products and for the preservation of liquid ophthalmological preparations have been discovered. These compositions contain sufficient disinfection activity, enabling a reasonable period of disinfection time and preserving efficacy, and they meet pharmacopoeia requirements for preservative efficacy.

The compositions are especially useful for disinfecting and/or cleaning of contact lenses; they contain an effective amount of an antimicrobial bioflavonoid, alone or in combination with ascorbic acid, and/or e.g. buffers, wetting agents, cleaning agents, tonicity agents, chelating agents, viscosity agents, counter-irritants. Also in combination with the other included substances, the eye compatibility is such, that there is little or no risk for eye irritation or other eye discomfort.

### Detailed description of the invention.

Flavonoids (mostly termed bioflavonoids) occur in all higher plants. They are neither present in bacteria, fungi and algae, nor in animals.

Bioflavonoids contain two C₆-rings, which are connected with each other by means of a C₃-bridge. The aromatic C₆-rings are substituted in many different ways, e.g. by hydroxylation, glycosylation and methylation. The C₃-ring is also substituted in several ways and a subdivision of the bioflavonoid group of compounds has been made on the basis of the latter phenomenon (a.o. flavanones, flavones, flavanonoles, flavonoles, proanthcyanidines). The structural formulas of the subtypes of bioflavonoids are based on the following

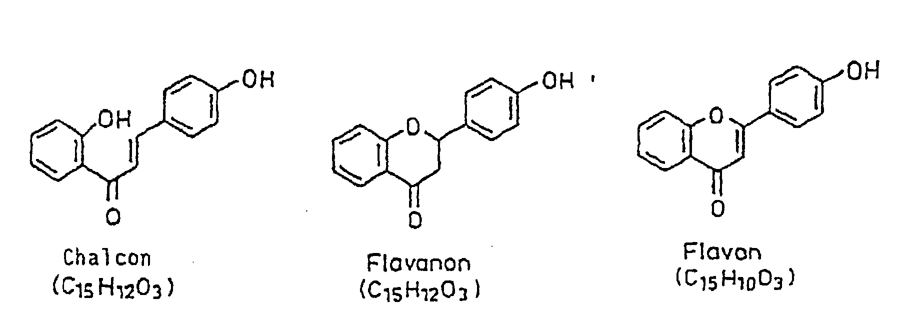

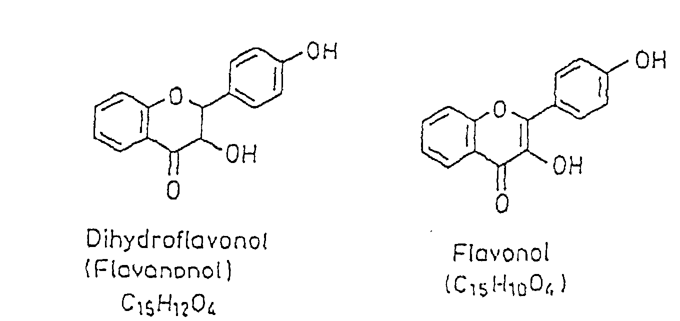

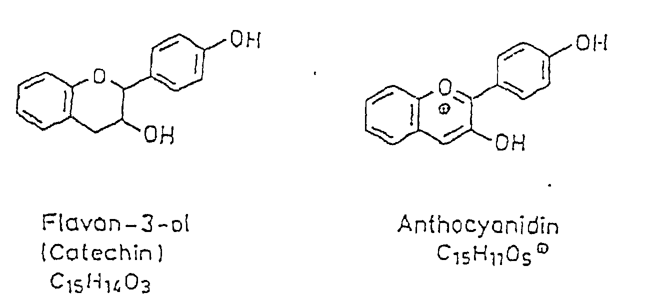

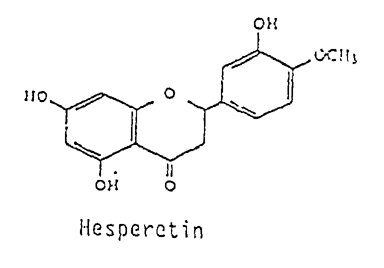

Bioflavonoids occur both as flavonoid glycocides (e.g. flavanone-glycosides; coumarin-glycosides) and as aglycones. The group of glycosylated bioflavonoids include e.g. hesperidin and naringin The corresponding aglycones are hesperetin and naringenin, respectively.

Far over 100 flavonoid glycosides and flavonoid aglycones are known. Most of them have been shown to have a certain degree of antimicrobial activity.

Flavonoids have metal-chelating properties; they can influence the availability of intracellular calcium, thereby interfering with the cyclical increase of free calcium during the cell cycle. This is particularly relevant in connection with rapidly proliferating cell systems, such as bacteria and tumor cells.

Flavonoids can also inhibit infection and replication of viruses, including influenza A and B, herpes, polio type 1 and respiratory syncytial viruses. In vitro, HIV is inactivated by bioflavonoids.

Ascorbic acid enhances the antiviral activity of flavones. Administration of ascorbic acid to individuals with herpes infections results in statistically significant clinical and antiviral effects. Ascorbic acid also enhances the activity of antibiotics against bacteria such as Staphylococcus aureus.

U.S. Patent 4.238.483 teaches a process for producing antimicrobial compositions from flavonoid glycosides. This process involves a heating step, which yields partically hydrolysed flavonoid compositions having antibacterial and antifungal activities. Said compositions are explicitly different from both flavonoid glycosides and from flavonoid aglycones.

U.S. Patent 5.425.944 describes an antimicrobial composition obtained after heating a grapefruit extract. The intended use of this composition is for antimicrobial preservation of food and for treatment of AIDS. There is no suggestion or indication concerning possible ophthalmological applications or any use in eye care products.

According to the present invention, bioflavonoids (with or without ascorbid acid) are used for the first time as an antimicrobial principle which can be used in the disinfection and preservation of eye care products and ophthalmological preparations.

Examples of useful bioflavonoid aglycones include, but are not limited to, hesperetin and naringenin. An example of a useful bioflavonoid glycoside is hesperedin. Bioflavonoid concentrations are preferably in the range of 0,0001% to 2% (by weight per volume of the total composition). More preferably the concentration range is 0,0001% to 0,5%.

The ophthalmologically useful compositions may contain other antimicrobial agents and adjuvants. These include, but are not limited to, ascorbic acid, sorbic acid and PHMB. In certain compositions, wetting and lubricating components are included in order to enhance contact lens wearing comfort. Such components include, but are not limited to, carboxymethylcellulose, hydroxymethylpropylcellulose, polyvinylalcohol, polyvinylpyrrolidine, polyoxamers, further acceptable cellulose derivatives and acceptable non-ionic surfactants and mixtures thereof. These components are present in a concentration range of 0,01% tot 4% (weight/volume) of the compositon.

For use in eye care products, additional components, conventionally used in this type of products, include buffering systems, cleaning substances, wetting agents, viscosity building compounds, tonicity agents and the like. They are used in concentrations similar to those used in conventional contact lens care products.

As additional compounds, certain enzymes capable of breaking down protein and lipid deposits can also be used. A preferred composition contains an amount of protein lipid and mucus degrading activity which is sufficient to remove debris from a lens due to normal wear, within a reasonable period of time. The useful enzymes include, but are not limited to, proteases, lipases and amylases and mixtures thereof.

A preferred composition for the one-step disinfection/cleaning of soft contact lenses contains an adequate amount of antimicrobial bioflavonoid, ascorbic acid, EDTA, a non-ionic detergent and a tonicity substance. More specifically, such a composition contains 0,0001% tot 0,5% bioflavonoid compound, 0,0001% tot 0,5% ascorbic acid, phosphate buffer (pH 7,4) 0,001% Na-EDTA, 0,2% poloxamer and 0,7% NaCl. Such a composition can also be provided in the form of a tablet which can be dissolved in a prescribed volume of water; the indicated percentages are on the basis of the required aqueous composition. Placing the contact lenses into this composition disinfects the lenses within six hours, and after this treatment the lenses can be brought back onto the eye without any further rinsing.

The method for disinfecting and/or cleaning of contact lenses by using the composition according to this invention is also effective against the unicellular organism Acanthamoeba, which can cause eye disease in individuals wearing soft contact lenses. Acanthamoeba is a species of unicellular organisms which are pathogenic to humans in that they cause severe infections of the eye.

In addition to the method for disinfecting/cleaning of contact lenses and the preservation of solutions for contact lens care, it is also possible to prepare eye drops and artificial tears on the basis of the compositions of this inventions. In this context, a preferred composition contains a sufficient amount of antimicrobial bioflavonoid (e.g. the aglycone hesperitin), a viscosity building substance, such as HPMC and/or PVP, an acceptable buffer system (e.g. phosphate buffer; pH 7,4) and NaCl sufficient to reach tonicity. Such a composition is suitable for use as an eye lubricant in "dry eye" condition (impaired production of tear fluid) and as wetting/storing solution for contact lenses.

In vivo studies on animals have shown that disinfection/cleaning and preserved artificial tear compositions according to the present invention are non-irritating to the eye and the skin; in cell culture studies, no cytotoxic effects were observed.

The following examples are given in order to illustrate, but not to limit, the scope of the present invention.

### Example 1.

A disinfecting/cleaning solution was prepared, containing a phosphate buffer (0,10% NaH₂PO₄.H₂O; 0,45% Na₂HPO₄.2H₂O; pH 7,4); 0,02% glycerol; 0,01% Na-EDTA; 0,2% Pluronic 127; 0,001 % ascorbic acid; 0,02% hesperetin and 0,7% NaCl. When tested on a panel of bacteria, fungi and yeasts, the following microbicidal effects were shown (data given as logarithmic values of the drop in numbers of microorganisms):

| Log. drops (microbicial effects) (* = total kill) | | | |
|---|---|---|---|
| | After 2 hrs. | After 4 hrs. | After 6 hrs. |
| Candida albicans | 3,6 | 7,3* | 7,3* |
| Escherichia coli | 9,6* | 9,6* | 9,6* |
| Pseudomonas aerigunosa | 3,5 | 8,5* | 8,5* |
| Staphylococcus aureus | 2,4 | 3,5 | 7,4* |
| Aspergillus niger | 3,9* | 3,9* | 3,9* |

This composition passes the U.S. and European Pharmacopea criteria for disinfecting contact lens solutions.

### Example 2.

The disinfecting/cleaning composition of Example 1 was tested for anti-Acanthamoeba activity. The unicellular parasite Acanthamoeba occurs in two forms: as the so-called trophozoite (actively living form) and as cyst (survival form under unfavourable conditions). The cysts are far more difficult to kill than the trophozoites. The composition of Example 1 was able to kill trophozoites within 30 to 60 minutes. The D-value for cysts (90% killing time) for cysts was 4 tot 6 hrs., depening on the Acanthamoeba subspecies. After a period of 24 hrs., all organisms had been killed.

### Example 3.

Another useful disinfecting composition contains 0,1% NaH₂PO₄.H₂O; 0,45% Na₂HPO₄.2H₂O; 0,003% glycerol; 0,5% NaCl; 0,02% Pluronic FI27; 0,002% ascorbic acid and 0,003% hesperidin. The following microbicidal effects were shown (data given as logarithmic values of the drop in numbers of microorganisms):

| Log. drops (microbicial effects) (* = total kill) | | | |
|---|---|---|---|
| | After 2 hrs. | After 4 hrs. | After 6 hrs. |
| Candida albicans | 1,3 | 3,0 | 6,9* |
| Escherichia coli | 3,5 | 3,8 | 7,6* |
| Pseudomonas aerigunosa | 3,8 | 4,1 | 7,3* |
| Staphylococcus aureus | 3,9 | 4,1 | 7,6* |
| Aspergillus niger | 0,6 | 1,4 | 4,4* |

This composition passes the U.S. and European Pharmacopea criteria for disinfecting contact lens solutions.

### Example 4.

A useful composition for the preservation of a lubricating solution for use as artificial tears (comfort drops) contains: 0,1% NaH₂PO₃.H₂O; 0,45% Na₂HPO₄.2H₂O; 0,02% glycerol; 0,01% Na-EDTA; 0,2% HPMC; 0,7% NaCl; 0,001% ascorbic acid and 0,02% hesperidin. After passage through a 200 micron filter this composition was aseptically transferred into plastic containers, closed and stored for 6 months. The containers were then opened and the lubricating solution was tested for antimicrobial efficacy. The results are shown below (data are given as logarithmic values of the drop in numbers of microorganisms):

| Log. drops (microbicial effects) (* = total kill) | | | |
|---|---|---|---|
| | After 1 wk. | After 2 wks. | After 4 wks. |
| Candida albicans | 7,3* | 7,3* | 7,3* |
| Escherichia coli | 9,6* | 9,6* | 9,6* |
| Pseudomonas aerigunosa | 3,3 | 3,4 | 3,2 |
| Staphylococcus aureus | 7,4* | 7,4* | 7,4* |
| Aspergillus niger | 3,9* | 3,9* | 3,9* |

This composition passes the U.S. and European Pharmacopea criteria for the preservation of contact lens care products.

### Example 5.

The composition of Example 4 was tested during 3 months in a contact lens practise on 320 individuals when fitting contact lenses. No signs of irritation or eye redness were notices. In an opthalmological practise the composition was tested as a comfort drop prior to eye examination (mydriasis or anaesthetic). In the 120 cases tested, no discomfort was observed by the opthalmologists or experienced by the patients.

### Example 6.

A stable and useful preserved composition for rinsing, conditioning and storing contact lenses includes a physiological saline solution (phosphate-buffered; pH 7,2), 0,01% Na-EDTA; 0,02% glycerol, 0,001% ascorbic acid and 0,02% hesperetin. Following the preparation (aseptic filling of a plastic container, after passing the solution through a 200 micron filter), the screw caps of the containers were removed; the open containers were then stored in a bathroom during 4 months. The antimicrobial activity of the preserved saline solution was tested.

The results were as follows (data are given as logarithmic values of the drops in numbers of microorganisms):

| Log. drops (microbicial effects) (* = total kill) | | | |
|---|---|---|---|
| | After 28 days | After 54 days | After 84 days |
| Candida albicans | 6,2* | 6,2* | 6,2* |
| Escherichia coli | 9,6* | 9,6* | 9,6* |
| Pseudomonas aerigunosa | 9,1* | 9,1* | 9,1* |
| Staphylococcus aureus | 8,8* | 8,8* | 8,8* |
| Aspergillus niger | 4,6* | 4,6* | 4,6* |

As can be seen, this composition is completely preserved for a period of at least 3 months (these values, obtained after a non-sterile period of 4 months, are identical to the values obtained with the freshly prepared composition).

### Example 7.

The preserved balanced salt solution of Example 6 was used to rinse patient conjunctiva to remove unattached foreign bodies, mucous debris (especially in most vulnerable cases such as patients with keratoconjunctivitis sicca with diffuse or focal corneal epithelial erosions) and to moisten fluorescein strips in order to stain the tear film. None of the, in excess of 200, patients treated or examined complained about any added discomfort to the eye by the composition other than the symptoms of the basic condition that was treated. In the patients on which the composition was used for diagnostic purposes and which had no corneal erosive diseases, no discomfort was reported at all.

### Example 8.

A preferred composition including, instead of hesperitin, narangenin, which is another bioflavonoid aglycone, and having the same composition as Example 1, gave microbicidal effects which are comparable to those shown in the table of Example 1.

## Claims

1. The use of a composition containing bioflavonoids for disinfecting and/or cleaning contact lenses.

2. Use according to claim 1, **characterised in that** the bioflavonoids are bioflavonoid aglycones.

3. Use according to claim 1, **characterised in that** the bioflavonoids are bioflavonoid glycosides.

4. Use according to claim 2, **characterised in that** hesperetin is present as a bioflavonoid aglycone compound.

5. Use according to claim 3, **characterised in that** hesperidin is present as bioflavonoid glycoside compound.

6. Use according to any one of claims 1 - 5, **characterised in that** the composition also contains ascorbic acid.

7. Use according to any one of claims 1 - 6, containing 0,0001 weight%- 0,5 weight% of the compounds having an antimicrobial activity, based on the total weight of the aqueous product.

8. Use according to claim 7, **characterised in that** the composition also contains 0,0001 weight% - 0,5 weight% ascorbic acid, based on the total weight of the aqueous product.

9. Use according to any one of claims 1 - 8, containing, apart from the antimicrobial compound, a buffer, EDTA, a viscosity increasing substance, and a compound for isotonicity.

10. Use according to any one of claims 1 - 8, containing, apart from the antimicrobial compound, a buffer, EDTA, a detergent, and a compound for isotonicity.

11. Use according to any one of claims 1 - 10, **characterised in that** it includes at least one enzyme capable of removing debris from a contact lens.

12. Use according to any one of claims 1 - 11, **characterised in that** the formulation is provided in the form of a tablet.

13. Use according to any one of claims 1 - 11, **characterised in that** the formulation is provided in the form of an aqueous solution.

## Patentansprüche

1. Verwendung einer Bioflavonoide enthaltenden Zusammensetzung zur Desinfektion und/oder Reinigung von Kontaktlinsen.

2. Verwendung nach Anspruch 1, dadurch charakterisiert, doss die Bioflavonoide Bioflavonoid-Aglycone sind.

3. Verwendung nach Anspruch 1, dadurch charakterisiert, dass die Bloflavonoide Bioflavonoid-Glycoside sind.

4. Verwendung nach Anspruch 2, dadurch charakterisiert, dass Hesperetin als eine Bioflavonoid-Aglycon-Verbindung vorliegt.

5. Verwendung nach Anspruch 3, dadurch charakterisiert, dass Hesperidin als Bioflavonoid-Glycosid-Verbindung vorliegt.

6. Verwendung nach einem der Anspruche 1 bis 5, dadurch charakterisiert, dass die Zusammensetzung auch Ascorbinsäure enthält.

7. Verwendung nach einem der Ansprüche 1 bis 6, enthaltend 0,0001 Gew.-% bis 0.5 Gew.-% der Verbindungen mit einer antimikrobiellen Aktivität bezogen auf das Gesamtgewicht des wässrigen Produktes.

8. Verwendung nach Anspruch 7, dadurch charakterisiert, dass die Zusammensetzung auch 0,0001 Gew.-% bis 0,5 Gew.-% Ascorbinsäure enthält bezogen auf das Gesamtgewicht des wässrigen Produktes.

9. Verwendung nach einem der Ansprüche 1 bis 8, enthatend abgesehen von der antimikrobiellen Verbindung einen Puffer, EDTA, eine viskositätserhöhende Substanz und eine Verbindung zur Isotonizität.

10. Verwendung nach einem der Ansprüche 1 bis 8, enthaltend abgesehen von der antimikrobiellen Verbindung einen Puffer, EDTA, ein Detergens und eine Verbindung zur Isotonizität.

11. Verwendung nach einem der Ansprüche 1 bis 10, dadurch charakterisiert, dass sie mindestens ein Enzym beinhaltet, das zur Entfernung von Ablagerungen von einer Kontaktlinse fähig ist.

12. Verwendung nach einem der Anspruche 1 bis 11, dadurch charakterisiert, dass die Formulierung in Form einer Tablette bereitgestellt wird.

13. Verwendung nach einem der Ansprüche 1 bis 11, dadurch charakterisiert, dass die Formulierung in Form einer wässrigen Lösung bereitgestellt wird.

## Revendications

1. L'utilisation d'une composition contenant des biflavonoïdes pour desinfection et/ou nettoyage de lentilles de contact.

2. Utilisation selon la revendications 1, **caractérisée en ce que** les bioflavonoïdes sont des aglycones de bioflavonoïde.

3. Utilisation selon la revendication 1, **caractérisée en ce que** les bioflavonoïdes sont des glycosides de bioflavonoïde.

4. Utilisation selon la revendication 2, **caractérisée en ce que** de l'hespéretine est présente en tant que composé de aglycone de bioflavonoïde.

5. Utilisation selon la revendications 3, **caractérisée en ce que** de l'hespéridine est présente en tant que composé glycoside de bioflavonoïde.

6. Utilisation selon une quelconque des revendications 1 à 5, **caractérisée en ce que** la composition contient également de l'acide ascorbique.

7. Utilisation selon une quelconque des revendications 1 à 6, contenant 0,0001% en poids à 0,5% en poids des composés présentant une activité anti-microbienne, sur la base du poids total du produit aqueux.

8. Utilisation selon la revendication 7, **caractérisé en ce que** composition contient également 0,0001% en poids à 0,5% en poids d'acide ascorbique, sur la base du poids total du produit aqueux.

9. Utilisation selon une quelconque des revendications 1 à 8 contenant, à part le composé anti-microbien, un tampon, de l'EDTA, une substance d'augmentation de viscosité, et un composé d'isotonicité.

10. Utilisation selon une quelconque des revendications 1 à 8, contenant, à part le composé anti-microbien, un tampon, de l'EDTA, un détergent et un composé d'isotonicité.

11. Utilisation selon une quelconque des revendications 1 à 10, **caractérisée en ce qu'**elle comprend au moins une enzime susceptible de retirer des détritus d'une lentille de contact.

12. Utilisation selon une quelconque des revendications 1 à 11, **caractérisée en ce que** la formulation est prévue sous la forme d'un comprimé.

13. Utilisation selon une quelconque des revendications 1 à 11, **caractérisée en ce que** la formulation est prévue sur la forme d'une solution aqueuse.
